Europäisches Patentamt

European Patent Office

Office européen des brevets

(11) Numéro de publication: **0 335 053**
A1

## (12) DEMANDE DE BREVET EUROPEEN

(21) Numéro de dépôt: 88420106.2

(22) Date de dépôt: 30.03.88

(51) Int. Cl.4: **B01F 1/00 , A61M 1/16**

(43) Date de publication de la demande:
04.10.89 Bulletin 89/40

(84) Etats contractants désignés:
AT BE CH DE ES FR GB GR IT LI LU NL SE

(71) Demandeur: SOCIETE DE MATERIELS ANNEXES DE DIALYSE S.M.A.D. (S.A. de droit francais)
88, Chemin de l'Aigas
F-69160 Tassin-la-Demi-Lune(FR)

(72) Inventeur: Weber, Daniel
La Clotte
F-47220 Astaffort(FR)

(74) Mandataire: Maureau, Philippe
Cabinet Germain & Maureau Le Britannia - Tour C 20, bld Eugène Déruelle Boîte Postale 3011
F-69392 Lyon Cédex 03(FR)

(54) Dispositif de préparation en continu de solutions de dialyse à partir d'un produit solide se présentant sous forme de granulés ou de poudre.

(57) Ce dispositif comprend une cuve de mélange (4) dans laquelle débouchent une tubulure (5) d'amenée d'eau traitée et une tubulure (6) de sortie du liquide de dialyse équipée d'un filtre (7), un dispositif d'agitation (15) du liquide contenu dans la cuve, un système d'alimentation (10) avec dosage en produit solide à partir d'une zone de stockage telle qu'une trémie (2), un système (8) de contrôle du niveau de liquide dans la cuve, et un système (9) de mesure de la concentration du dialysat, des moyens étant prévus qui assurent la circulation de liquide et l'amenée des produits solides dans la cuve en tenant compte du temps de séjour des produits solides dans la cuve, du temps de dissolution des produits solides, du débit réel de distribution des produits solides dans la cuve et du débit théorique d'amenée des produits solides dans la cuve.

FIG.2

Xerox Copy Centre

## Dispositif de préparation en continu de solutions de dialyse à partir d'un produit solide se présentant sous forme de granulés ou de poudre

La présente invention a pour objet un dispositif de préparation en continu de solutions de dialyse à partir d'un produit solide se présentant sous forme de granulés ou de poudre.

L'insuffisance rénale chronique touche environ 200 000 personnes dans le monde dont l'épuration du sang est assurée par la technique de dialyse, de manière définitive ou provisoire dans l'attente d'une transplantation.

Lors d'une opération de dialyse, le sang du patient est épuré dans un rein artificiel où circule une solution de dialyse ou dialysat dont la composition électrolytique est voisine de celle du plasma sanguin. Une solution de dialyse comprend une teneur élevée en sodium, en acétate ou en bicarbonate, (suivant qu'il s'agit d'un dialysat à l'acétate ou d'un dialysat au bicarbonate), en chlorures, et dans des proportions moindres d'autres ions tels que Calcium, Magnésium et Potassium. Au cours de trois séances de dialyse hebdomadaire, chaque patient consomme de 120 à 200 litres de dialysat par séance.

Ces volumes importants de dialysat sont actuellement préparés en continu au pied du lit du patient à l'aide d'un appareil dénommé générateur de dialyse, assurant la dilution d'un liquide concentré dans de l'eau, dans des proportions d'un volume de concentré pour 34 volumes d'eau. Pour une dialyse traditionnelle à l'acétate, il convient de disposer de 5 litres de concentré, tandis que pour une dialyse au bicarbonate, il convient de disposer de deux concentrés, a savoir 5 litres de concentré acide et 7 litres de de concentré de bicarbonate, ces deux éléments devant être mélangés au dernier moment, du fait qu'ils ne peuvent pas être conservés en mélange sous une forme stable.

Pour les concentrés de bicarbonate, cette technique pose de nombreux problèmes, et notamment celui de la conservation des liquides concentrés qui doit être effectuée dans des conditions de température strictes et qui ne peut être assurée que pendant des périodes relativement brèves, de l'ordre d'un mois. En outre, ces concentrés liquides nécessitent la manipulation de masses importantes, puisque chaque dialysé consomme, suivant le type de dialyse, entre 750 et 1 800 litres de concentré par an. Enfin, les générateurs de dialyse traditionnels destinés à la préparation d'un liquide de dialyse à l'acétate, ne conviennent pas pour la préparation d'un liquide de dialyse au bicarbonate.

Ces différentes contraintes limitent considérablement l'expansion de la dialyse au bicarbonate, et notamment la mise en oeuvre de ce type de dialyse au domicile du patient.

Il existe également des centrales de préparation de liquide de dialyse, destinées à l'alimentation simultanée de plusieurs patients, qui fonctionnent suivant le même principe.

Il a été imaginé de réaliser à partir de produits solides, le liquide concentré mis en oeuvre dans les techniques définies précédemment. Cette solution présente l'avantage de réduire le volume et les masses transportées, et de permettre l'utilisation des appareils existants. Elle présente, toutefois, l'inconvénient de nécessiter une phase supplémentaire dans le cycle de préparation du liquide de dialyse, et augmente donc les risques d'erreur lors de la préparation du concentré.

La présente invention vise à remédier à ces inconvénients en fournissant un dispositif permettant la fabrication de liquides de dialyse, en continu, à partir de produits solides, ce dispositif pouvant préparer indifféremment une solution de dialyse à base d'acétate ou une solution de dialyse à base de bicarbonate.

A cet effet, le dispositif qu'elle concerne comprend une cuve de mélange dans laquelle débouchent une tubulure d'amenée d'eau traitée et une tubulure de sortie du liquide de dialyse équipée d'un filtre, un dispositif d'agitation du liquide contenu dans la cuve, un système d'alimentation avec dosage en produits solides à partir d'une une de stockage telle qu'une trémie, un système de contrôle du niveau de liquide dans la cuve, et un système de mesure de la concentration du dialysat, des moyens étant prévus qui assurent la circulation de liquide et l'amenee des produits solides dans la cuve en tenant compte du temps de séjour des produits solides dans la cuve, du temps de dissolution des produits solides, du débit réel de distribution des produits solides dans la cuve et du débit théorique d'amenée des produits solides dans la cuve.

Ces différentes caractéristiques permettent la réalisation d'un dispositif de structure simple tout en assurant que le liquide de dialyse possède les propriétés requises. Cet appareil permettant une dilution directe des produits solides pour former le liquide de dialyse évite toutes les contraintes des concentrés liquides, telles que limite de solubilité et stabilité des constituants. Une dialyse nécessitant une consommation en produits solides de l'ordre de 300 g/heure, qu'il s'agisse de réaliser un liquide au bicarbonate ou un liquide à l'acétate, le même appareil peut donc être utilisé quel que soit le type de liquide de dialyse souhaité.

Avantageusement, les moyens de commande de l'amenée des produits solides et d'eau dans la cuve sont agencés de façon telle que les variations de la concentration du liquide de dialyse soient inférieures à 3,5 % par rapport à la concentration souhaitée, tandis que le rapport A du temps de séjour TS des produits solides dans la cuve et du temps de dissolution TD des produits solides est inférieur à 20, et que le rapport B du débit réel Q1 de distribution des produits solides et du débit souhaité Q0 de distribution des produits solides est compris entre 1 et 3, le débit souhaité Q0 de distribution des produits solides étant égal au rapport du produit de la concentration souhaitée C0 en dialysat par le volume V de la cuve de dissolution et du temps de séjour TS du liquide dans la cuve.

Le respect du rapport des concentrations entre les différents constituants ne dépend, lui, que de la qualité du mélange solide, c'est-à-dire de l'homogénéité de celui-ci. Le degré d'homogénéité du produit doit être d'autant plus important que le volume de la cuve est faible. Ainsi, pour une cuve de préparation de 1 litre, d'un liquide concentré à 10 g/l, l'homogénéité devra être respectée sur un échantillon de 10 g de produit solide (selon normes de la Pharmacopée concernant les liquides de dialyse).

Selon une autre caractéristique de l'invention, les moyens de commande de l'amenee de produits solides sont constitués par une sonde mesurant la conductivité du dialysat et permettant d'asservir le fonctionnement du système d'alimentation en produits solides à la concentration du dialysat.

Il est également possible d'assurer le maintien de la concentration à une valeur constante en plaçant un débitmètre sur le conduit de sortie du dialysat, qui commande les moyens d'amenee en produits solides. Il est à noter que, pour des débits constants d'amenée de produits solides et de dialysat, la concentration de la solution demeure constante.

Conformément à d'autres particularités de l'invention, la trémie de stockage des produits solides a un volume de 2 à 5 litres, le système de dosage et d'alimentation en produits solides a un débit de 1 à 30 g/mn, et la cuve de dissolution possède un volume de 0,1 à 2 litres.

Selon une première forme d'exécution de ce dispositif, celui-ci comprend une cuve à l'air libre, et le conduit d'amenee d'eau est équipé d'une vanne dont l'ouverture est commandée par le détecteur de niveau, tandis que le conduit de sortie du liquide de dialyse est équipé d'une pompe de remise en pression, à une valeur de l'ordre de 500 millibars (1 bar = 10⁵ Pa), et un débit de l'ordre de 0,1 à 1 litre/mn.

Selon une autre forme d'exécution de ce dispositif, l'intérieur de la cuve est isolé du milieu extérieur et une tubulure débouchant dans la cuve au-dessus du niveau de liquide est équipée d'une pompe qui, susceptible d'alimenter la cuve en air ou en gaz neutre avec un débit de 0,1 à 10 cm³/mn, ou de réaliser une aspiration à l'intérieur de celle-ci, est commandée par le détecteur de niveau.

Conformément à une première possibilité, dans ce cas, la trémie de stockage des produits solides est équipée d'un couvercle de fermeture étanche et les moyens d'actionnement du système d'alimentation en produits solides, constitué par exemple par une vis sans fin, sont également étanches.

Conformément à une deuxième possibilité, dans ce cas, la trémie de stockage des produits solides est à l'air libre et les moyens d'alimentation de la cuve en produits solides sont constitués par un système de dosage étanche, tel qu'une écluse pivotante, assurant en permanence une séparation avec étanchéité de la trémie et de la cuve.

Le dispositif d'agitation à l'intérieur de la cuve peut être constitué par un agitateur magnétique ou à hélice ou à recirculation, ou bien la cuve peut possèder la forme d'un tuyau vertical dans le fond duquel est amenée l'eau de dissolution par un orifice rétréci et dans le haut duquel tombent les granulés. Cette dernière solution présente l'avantage de fournir un dispositif d'agitation intégré, dont l'efficacité peut être renforcée par un système à recirculation du dialysat.

De toute façon, l'invention sera bien comprise à l'aide de la description qui suit, en référence au dessin schématique annexe représentant, à titre d'exemples non limitatifs, plusieurs formes d'exécution de ce dispositif :

Figure 1 est une vue en coupe d'un dispositif très schématique illustrant le principe de fonctionnement de celui-ci ;

Figures 2, 3 et 5 représentent trois formes d'exécution de ce dispositif vu en coupe ;

Figure 4 est une vue partielle d'une variante du dispositif de figure 3.

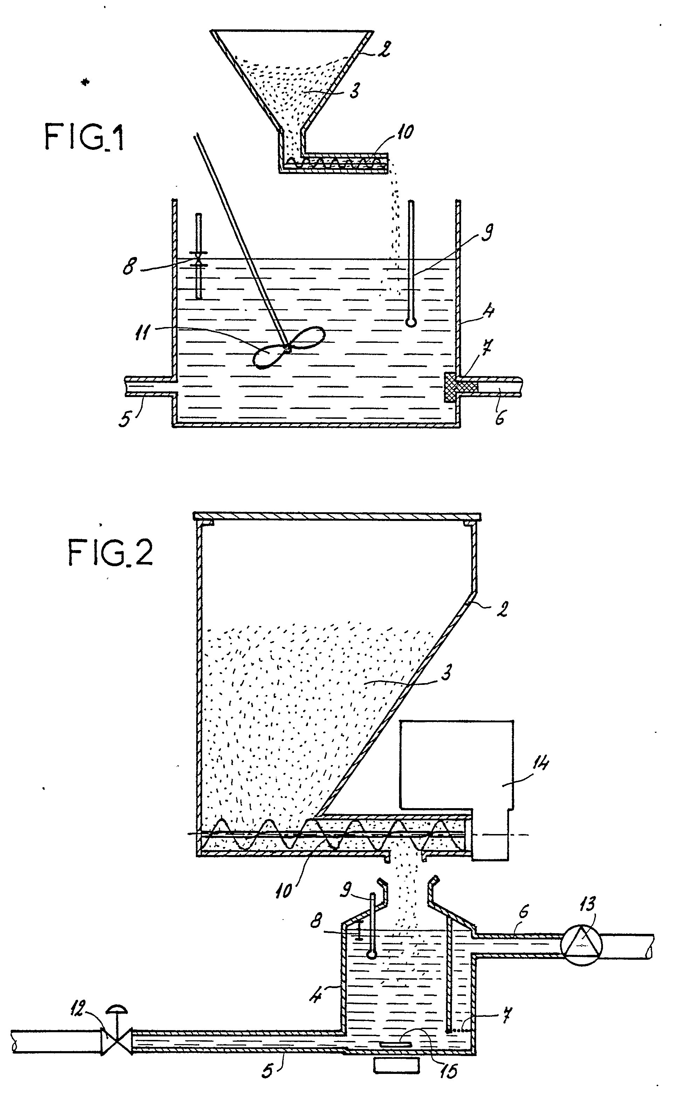

Le dispositif selon l'invention comprend, de façon générale et comme montré à la figure 1, une trémie 2 de stockage de produits solides tels que des granulés 3, d'un volume de 1 à 5 litres, et une cuve 4 de dissolution des granulés dans de l'eau. Cette cuve 4 est équipée d'un conduit 5 d'amenée de l'eau traitée et d'un conduit 6 d'évacuation du liquide de dialyse, un filtre 7 étant disposé immédiatement en aval de l'orifice de sortie du liquide de dialyse, dont la maille, inférieure à 0,1 mm, empêche l'évacuation des produits non dissous. La cuve 4, d'un volume de 0,1 à 2 litres, est équipée d'un détecteur 8 du niveau de liquide, ainsi que d'une sonde 9 mesurant la concentration du liquide de dialyse, par exemple par conductivité. L'amenée des granulés 3 dans la cuve 4 avec dosage de ceux-ci est réalisée par un dispositif de type connu constitué, par exemple, par une vis sans fin 10. Un agitateur 11 favorise la dissolution des granulés et assure l'homogénéisation de la solution.

Dans la forme d'exécution représentée à la figure 2 dans laquelle les mêmes éléments sont désignés par les mêmes références que précédemment, le conduit 5 d'amenée d'eau est équipé d'une vanne 12, et le conduit 6 d'évacuation du liquide de dialyse est équipé d'une pompe 13 de remise en pression. Le détecteur de niveau 8 agit sur la vanne 12 pour assurer le maintien du niveau de liquide à la valeur de consigne, tandis que la sonde 9 de mesure de la conductivité agit sur le moteur 14 entraînant la vis sans fin 10 en vue de l'amenée des granulés dans la cuve. Dans cette forme d'exécution, l'agitation est réalisée par un agitateur magnétique 15.

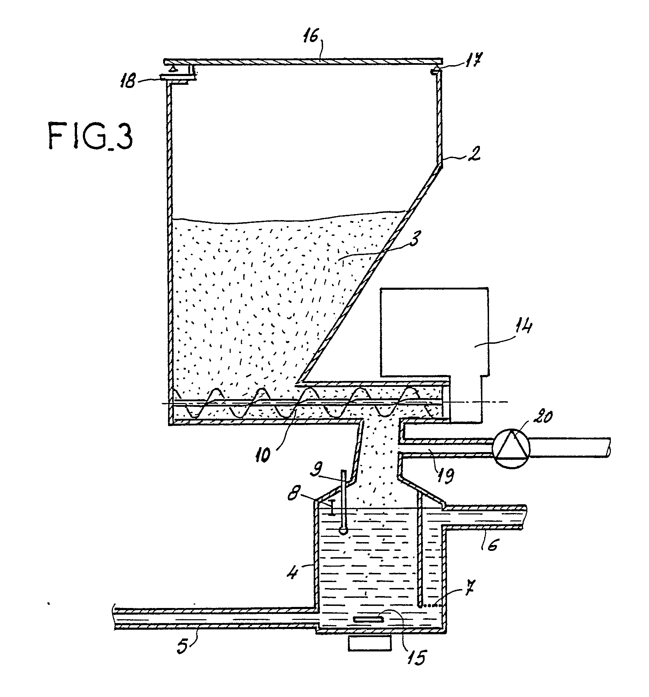

Dans la forme d'exécution représentée à la figure 3, dans laquelle les mêmes références désignent les mêmes éléments que précédemment, le dispositif est étanche. A cet effet, la trémie 2 est fermée par un couvercle 16, avec interposition de joints 17, un contact 18 permettant de vérifier l'état de fermeture. Le dispositif d'entraînement 14 de la vis sans fin 10 est également étanche. En outre, dans la partie superieure de la cuve 4, qui est reliée de façon étanche à l'orifice de sortie de la vis sans fin 10, débouche une tubulure 19 sur laquelle est montée une pompe 20, destinée à introduire dans la cuve ou extraire de celle-ci de l'air. La pompe 20 est commandée par le détecteur de niveau 8 de telle sorte que la pression régnant au-dessus du liquide contenu dans la cuve assure un maintien de ce niveau a une valeur constante.

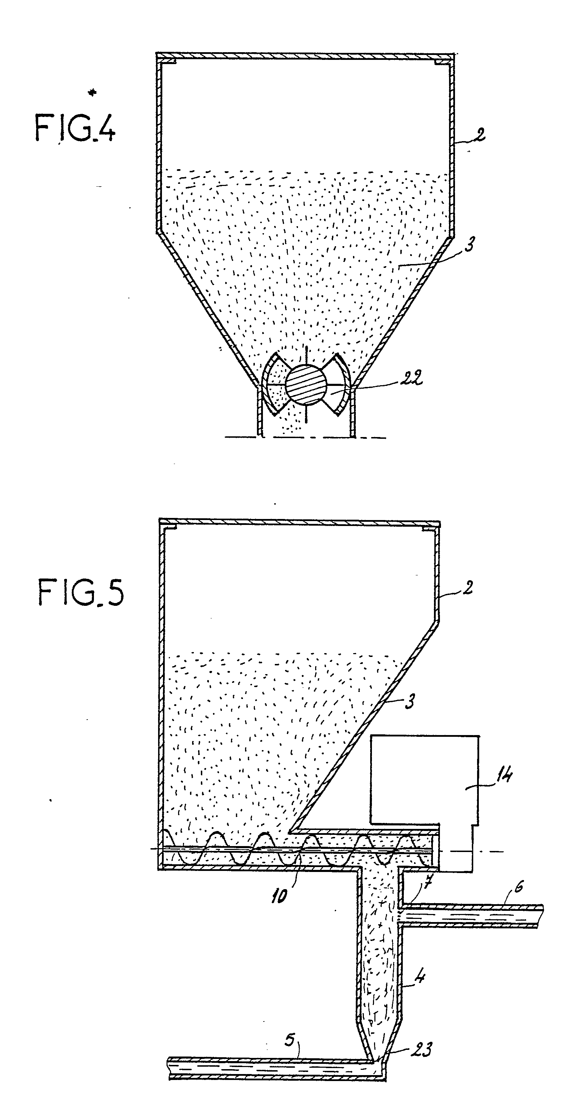

La figure 4 représente une variante du dispositif de figure 3 dans laquelle la trémie 2 n'est pas fermée de façon étanche, mais dans laquelle le dispositif de transfert et de dosage permettant le passage des granulés dans la cuve assure en permanence une séparation étanche entre la trémie et la cuve. A cet effet, le dispositif de transfert peut être constitué par exemple par une écluse pivotante telle que celle 22 représentée au dessin.

La figure 5 représente une variante d'exécution pouvant être appliquée à l'un quelconque des dispositifs des figures 2 à 4, selon laquelle la cuve 4 se présente sous la forme d'un tube allongé disposé verticalement, dans lequel l'eau est amenée en partie basse par un orifice 23 de section réduite et dans lequel les granulés sont amenés en partie haute. Le contre-courant assuré entre l'eau et les granulés assure une parfaite dissolution de ceux-ci sans nécessiter la mise en oeuvre d'un agitateur spécifique.

Comme il ressort de ce qui précède, l'invention apporte une grande amélioration à la technique existante en fournissant un dispositif permettant la préparation en continu de solutions de dialyse à partir de produits concentrés granulés.

Comme il va de soi, l'invention ne se limite pas aux seules formes d'exécution de ce dispositif décrites ci-dessus à titre d'exemples elle en embrasse, au contraire, toutes les variantes de réalisation. C'est ainsi notamment que ce dispositif pourrait tout aussi bien convenir pour la préparation de dialysat pour plusieurs patients, sous réserve d'un dimensionnement différent de ses principaux organes, le volume de la trémie pouvant être de l'ordre de 200 litres, celui de la cuve de 5 à 100 litres, et le débit d'amenée des produits solides de l'ordre de 20 à 400 g/mn, ou encore que les produits solides pourraient être constitués, non pas par des granulés, mais par de la poudre, sans que l'on sorte pour autant du cadre de l'invention.

**Revendications**

1- Dispositif de préparation en continu de solutions de dialyse à partir de produits solides se présentant sous forme de granulés ou de poudre, caractérisé en ce qu'il comprend une cuve de mélange (4) dans laquelle débouchent une tubulure (5) d'amenée d'eau traitée et une tubulure (6) de sortie du liquide de dialyse équipée d'un filtre (7), un dispositif d'agitation (15) du liquide contenu dans la cuve, un système d'alimentation (10) avec dosage en produit solide à partir d'une zone de stockage telle qu'une trémie (2), un système (8) de contrôle du niveau de liquide dans la cuve, et un système (9) de mesure de la concentration du dialysat, des moyens étant prévus qui assurent la circulation de liquide et l'amenée des produits solides dans la cuve en tenant compte du temps de séjour des produits solides dans la cuve, du temps de dissolution des produits solides, du débit réel de distribution des produits solides dans la cuve et du débit théorique d'amenée des produits solides dans la cuve.

2- Dispositif selon la revendication 1, caractérisé en ce que les moyens de commande de l'amenée de produits solides et d'eau dans la cuve sont agencés de façon telle que les variations de la concentration du liquide de dialyse soient inférieures à 3,5 % par rapport à la concentration souhaitée, tandis que le rapport (A) du temps de séjour (TS) des produits solides dans la cuve et du temps de dissolution (TD) des produits solides est inférieur à 20, et que le rapport (13) du débit réel (Q1) de distribution des produits solides et du débit souhaité (Q0) de distribution des produits solides est compris entre 1 et 3, le débit souhaité (Q0) de distribution des produits solides étant égal au rapport du produit de la concentration souhaitée (C0)

en dialysat par le volume (V) de la cuve de dissolution, et du temps de séjour (TS) du liquide dans la cuve.

3- Dispositif selon l'une quelconque des revendications 1 et 2, caractérisé en ce que les moyens de commande de l'amenée des produits solides sont constitués par une sonde (9) mesurant la concentration du dialysat et permettant d'asservir le fonctionnement du système d'alimentation (10) en produits solides à la concentration du dialysat.

4- Dispositif selon l'une quelconque des revendications 1 et 2, caractérisé en ce qu'il comprend un débitmètre monté sur le conduit de sortie du dialysat et agissant sur les moyens de commande de l'amenée des produits solides, pour maintenir constante la concentration du dialysat.

5- Dispositif selon l'une quelconque des revendications 1 à 4, caractérisé en ce que la trémie (2) de stockage des produits a un volume de 1 à 5 litres, le système (10) de dosage et d'alimentation en produits a un débit de 1 à 20 g/mn, et la cuve (4) de dissolution possède un volume de 0,1 à 2 litres.

6- Dispositif selon l'une quelconque des revendications 1 à 4, caractérisé en ce que, dans le cas où il est destiné à la préparation de dialysat, simultanément pour plusieurs patients, la trémie de stockage des produits solides a un volume de l'ordre de 200 litres, le système de dosage et d'alimentation en produits solides a un débit de 20 à 400 g/mn et la cuve de dissolution possède un volume de 5 à 100 litres.

7- Dispositif selon l'une quelconque des revendications 1 à 6, caractérisé en ce qu'il comprend une cuve (4) à l'air libre, et le conduit (5) d'amenée d'eau est équipé d'une vanne (12) dont l'ouverture est commandée par le détecteur de niveau (8), tandis que le conduit (6) de sortie du liquide de dialyse est équipé d'une pompe (13) de remise en pression, à une valeur de l'ordre de 500 millibars (1 bar = $10^5$ Pa), et un débit de l'ordre de 0,1 à 1 litre/mn.

8- Dispositif selon l'une quelconque des revendications 1 à 6, caractérisé en ce que l'intérieur de la cuve (4) est isolé du milieu extérieur, et une tubulure (19) débouchant dans la cuve au-dessus du niveau de liquide est équipée d'une pompe (20) qui, susceptible d'alimenter la cuve en air ou en gaz neutre avec un débit de 0,1 à 10 cm$^3$/mn, ou de réaliser l'aspiration à l'intérieur de celle-ci, est commandée par le détecteur de niveau (8).

9- Dispositif selon la revendication 8, caractérise en ce que la trémie (2) de stockage des produits solides est équipée d'un couvercle de fermeture étanche (16) et les moyens d'actionnement du système d'alimentation en produits solides, constitué par exemple par une vis sans fin, sont également étanches.

10- Dispositif selon la revendication 8, caractérisé en ce que la trémie (2) de stockage des produits solides est à l'air libre et les moyens d'alimentation de la cuve en produits sont constitués par un système de dosage étanche tel qu'une écluse pivotante (22) assurant en permanence une séparation avec étanchéité de la trémie (2) et de la cuve (4).

11- Dispositif selon l'une quelconque des revendications 1 à 10, caractérisé en ce que la cuve (4) possède la forme d'un tuyau vertical dans le fond duquel est amenée l'eau de dissolution par un orifice rétréci (23) et dans le haut duquel tombent les produits solides.

FIG.1

FIG.2

FIG_3

FIG.4

FIG.5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| E | FR-A-2 604 922 (SOCIETE DE MATERIELS ANNEXES DE DIALYSE S.M.A.D.) * document complet * | 1-11 | B 01 F  1/00 A 61 M  1/16 |
| X | DE-A-1 903 027 (BOEHRINGER MANNHEIM) * page 3, lignes 8-28; pages 6-7 * | 1,3 | |
| A | | 7 | |
| A | FR-A-2 502 960 (TERSTEEGEN BERND) * page 7, lignes 1-17 * | 1 | |
| A | US-A-3 607 105 (REID) * colonne 1, lignes 48-75; colonnes 2-4 * | 1,7 | |
| A | FR-A-2 523 406 (FRERES) * page 7, lignes 4-9 * | 4 | |
| A | EP-A-0 033 374 (AGFA-GEVAERT) * page 11, lignes 10-11; pages 22-23 * | 5,8-10 | |
| A | GB-A-2 048 107 (KOREHA KAGAKU KOGYO) * pages 1-5 * | 8,9,11 | DOMAINES TECHNIQUES RECHERCHES (Int. Cl.4) B 01 F B 01 D A 61 M |
| A | US-A-3 539 539 (GOETZKE) * figure 3 * | 10,11 | |
| A | US-A-3 343 919 (MILLER) * colonne 2, lignes 55-72; colonnes 3-6 * | 11 | |

Le présent rapport a été établi pour toutes les revendications

| Lieu de la recherche | Date d'achèvement de la recherche | Examinateur |
|---|---|---|
| BERLIN | 13-10-1988 | CORDERO ALVAREZ M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)